# EUROPEAN PATENT SPECIFICATION

(11) **EP 1 761 537 B1**
(45) Date of publication and mention of the grant of the patent: **03.08.2011**
(21) Application number: 04745134.9
(22) Date of filing: 30.06.2004
(51) Int. Cl.: C07D 487/04

(54) **PYRROLO[2,1-C][1,4]BENZODIAZEPINE-NAPTHALIMIDE CONJUGATES LINKED THROUGH PIPERAZINE MOIETY AND PROCESS FOR PREPARATION THEREOF**
ÜBER DIE PIPERAZINEINHEIT GEBUNDENE PYRROLO[2,1-C][1,4]BENZODIAZEPIN-NAPTHALIMID-KONJUGATE UND VERFAHREN ZU DEREN HERSTELLUNG
CONJUGUES DE PYRROLO[2,1-C][1,4]BENZODIAZEPINE-NAPTHALIMIDE LIES PAR UNE FRACTION PIPERAZINE ET LEURS PROCEDES DE PREPARATION

(43) Date of publication of application: 14.03.2007
(73) Proprietor: Council of Scientific and Industrial Research, New Delhi 110 001 (IN)
(72) Inventor: KAMAL, A., c/o Indian Institute of Chemical Tech., Andhra Pradesh (IN); RONDLA, R., c/o Indian Institute of Chemical Tech., Andhra Pradesh (IN); KHANNA, G. B. R., c/o Indian Inst. of Chem. Tech., Andhra Pradesh (IN)
(74) Representative: Manaton, Ross Timothy
(86) International application number: PCT/IN2004/000191
(87) International publication number: WO 2006/003670

(56) References cited:
- WO-A-01/66545
- WO-A-94/07862
- US-A- 4 874 863
- KAMAL AHMED ET AL: "Design and synthesis of C-8 linked pyrrolobenzodiazepine-naphthalimide hybrids as anti-tumour agents" BIOORGANIC AND MEDICINAL CHEMISTRY LETTERS, vol. 12, no. 15, 5 August 2002 (2002-08-05), pages 1933-1935, XP002316525 ISSN: 0960-894X
- KAMAL A ET AL: "RECENT DEVELOPMENTS IN THE DESIGN, SYNTHESIS AND STRUCTURE-ACTIVITY RELATIONSHIP STUDIES OF PYRROLOÄ2,1-cÜÄ1,4ÜBENZODIAZEPINES AS DNA-INTERACTIVE ANTITIMOUR ANTIBIOTICS" CURRENT MEDICINAL CHEMISTRY. ANTI-CANCER AGENTS, BENTHAM SCIENCE PUBLISHERS, HILVERSUM, NL, vol. 2, no. 2, 2002, pages 215-254, XP008038559 ISSN: 1568-0118
- KAMAL AHMED ET AL: "Synthesis of C-8 alkylamino substituted pyrrolo(2,1-c)(1,4)benzodiaze pines as potential anti-cancer agents" BIOORGANIC AND MEDICINAL CHEMISTRY LETTERS, vol. 12, no. 15, 5 August 2002 (2002-08-05), pages 1917-1919, XP002316526 ISSN: 0960-894X

## Description

### Field of the invention

The present invention relates to novel pyrrolo[2,1-c][1,4]benzodiazepine-napthalimide hybrids linked through piperazine moiety as potential antitumour agents. The present invention also relates to a process for the preparation of novel pyrrolo[2,1-c][1,4]benzodiazepine-napthalimide hybrids linked through piperazine moiety useful as potential antitumour agents.

The present invention particularly relates to the synthesis of pyrrolo[2,1-c][1,4]benzodiazepine-napthalimide hybrids linked through piperazine moiety as possible anticancer agents. The structural formula of novel pyrrolo[2,1-c][1,4]benzodiazepine-napthalimide hybrids (VIII) is as follows, wherein n₁ = 2, 3, 4, n₂ = 2, 3, 4.

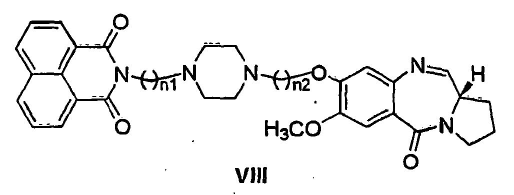

### Background of the invention

Pyrrolo[2,1-*c*][1,4]benzodiazepines are a family of DNA interactive antitumour antibiotics derived from *Streptomyces* species. Examples of naturally occurring pyrrolo[2,1-*c*][1,4]benzodiazepines include anthramycin, tomaymycin, sibiromycin and DC-81. These compounds show their biological activity through covalent binding via their N10-C11 imine/carbinol amine moiety to the C2-amine position of a guanine residue within the minor groove of DNA giving rise to the preference for Pu-G-Pu sequences. (Kunimoto, S.; Masuda, T.; Kanbayashi, N.; Hamada, M.; Naganawa, H.; Miyamoto, M.; Takeuchi, T and Unezawa, H. J. Antibiot., 1980, 33, 665.; Kohn, K. W. and Speous, C. L. J. Mol. Biol., 1970, 91, 551.; Hurley, L. H.; Gairpla, C. and Zmijewski, M. Biochem. Biophy. Acta., 1977, 475, 521.; Kaplan, D. J. and Hurley, L. H. Biochemistry, 1981, 20, 7572.) The molecules have a right-handed twist, when viewed from the C-ring towards the A-ring. This enables the PBD to mirror the curvature of B-form DNA and maintain isohelical contact with the walls and floor of the minor groove. In the last few years a growing interest has been shown in the development of new pyrrolo[2,1-*c*][1,4]benzodiazepine hybrids. Many PBD conjugates have been synthesized and investigated for their anticancer activity (Thurston, D. E.; Morris, S. J.; Hartley, J. A. Chem. Commun. 1996, 563.; Damayanthi, Y.; Reddy, B. S. P.; Lown, J. W. J. Org. Chem. 1999, 64, 290.; Kamal, A.; Reddy, B. S. N.; Reddy, G. S. K.; Ramesh, G Bioorg. Med. Chem. Lett. 2002, 12, 1933, Kamal, A.; Reddy, B. S. N.; Reddy Indian patent application No.209/DEL/2000). Recently C-8 linked PBD dimers with C2/C2 exounsaturation have been designed and synthesized (Gregson, S. J.; Howard, P. W.; Hartley, J. A.; Brooks, N. A.; Adam, L. J.; Jenkins, T. C.; Kelland, L. R and Thurston, D. E., J. Med. Chem. 2001, 44, 737). Also, non cross-linking mixed imine-amide PBD dimers have been synthesized that have significant DNA binding ability and potent antitumor activity (Kamal, A.; Ramesh, G.; Laxman, N.; Ramulu, P.; Srinivas, O.; Neelima, K.; Kondapi, A. K.; Srinu, V. B.; Nagarajaram, H. M. J. Med. Chem. 2002, 45, 4679). During earlier studies in this laboratory PBDs have been linked to naphthalimides through alkane chain which have shown promising anticancer activity (Kamal, A.; Reddy, B. S. N.; Reddy, G. S. K.; Ramesh, G Bioorg. Med Chem. Lett. 2002, 12, 1933, Kamal, A.; Reddy, B. S. N.; Reddy Indian patent application No.209/DEL/2000). However, in the present invention the PBD and naphthalimide moieties have been linked through piperazine moiety with alkyl side arms, instead of simple alkane chain spacers. By incorporation of a piperazine moiety in the spacer these new hybrids not only exhibit enhanced in vitro anticancer activity but remarkable DNA binding affinity for a number of this type of hybrids as illustrated in Table 1 and 2.

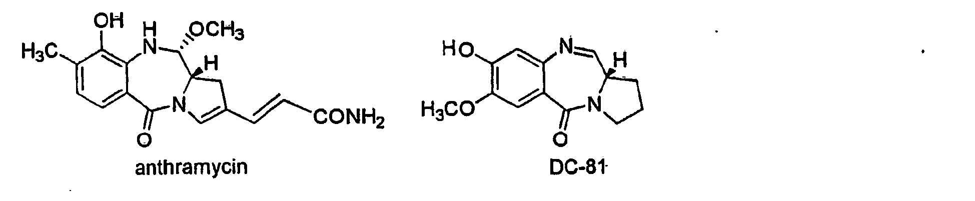

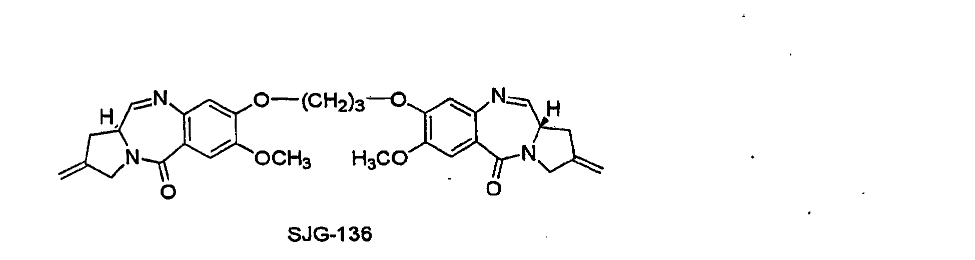

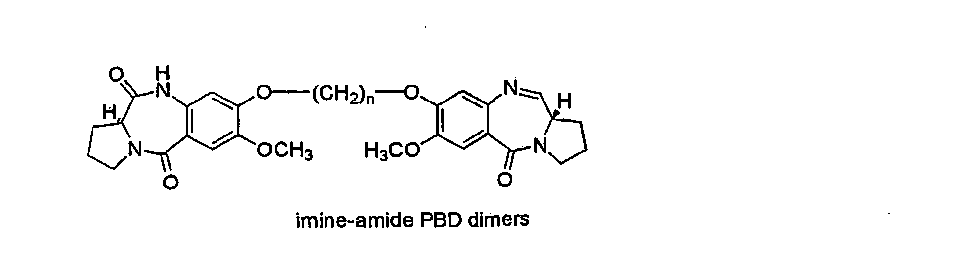

### Objects of the invention

The main objective of the present invention is to provide new pyrrolo[2,1-*c*][1,4]benzodiazepines useful as anticancer agents. Another objective of the present invention is to provide a process for the preparation of novel pyrrolo[2,1-*c*][1,4]benzodiazepihes useful as antitumor agents.

### Summary of the invention

Accordingly, the present invention provides a novel pyrrolo[2,1-*c*][1,4]benzodiazepines of formula VIII where n₁= 2, 3, 4, n₂ = 2, 3, 4.

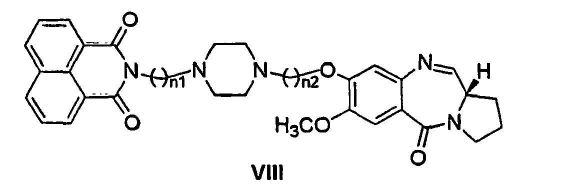

The present invention also provides a process for the preparation of pyrrolo[2,1-*c*][1,4]benzodiazepines of formula VIII shown above where n₁ = 2, 3, 4, n₂ = 2, 3, 4, which comprises of reacting (2S)-N-[4-hydroxy-5-methoxy-2-nitrobenzoyl]pyrrolidine-2-carboxaldehyde diethyl thioacetal of formula IV with dibromoalkanes in an aprotic water miscible organic solvent in the presence of a mild inorganic base at refluxing temperature for a period of 48h, isolating 2S-N-[4-(n-bromo alkoxy)-5-methoxy-2-nitrobenzoyl]pyrrolidine-2-carbaxaldehyde diethyl thioacetal of formula V, reacting the compound of formula V with piperazine attached naphthalimide in presence of mild inorganic bases isolating compound of formula VI, reducing it with SnCl₂.2H₂O in presence of organic solvent at a reflux temperature, reacting the above amino compound of formula VII with known deprotecting agents in a conventional manner to give novel pyrrol[2,1-*c*][1,4]benzodiazepine of formula VIII wherein n is as stated above.

### Detailed description of the invention

The precursor, (2S)-N-[4-hydroxy-5-methoxy-2-nitrobenzoyl]pyrrolidine-2-carboxaldehyde diethylthioacetal of formula IV (Thurston, D. E.; Murthy, V. S.; Langley, D. R.; Jones, G.; B. Synthesis, 1990, 81) has been prepared by literature methods.

Some representative compounds of formula VIII of present invention are given below:
1. 7-Methoxy-8-{2-[4-[2-(1,3-dioxo-benz[*de*]isoquinolin-2-yl)ethyl]piperazin-1=yl]ethyl}-oxy-(11aS)-1,2,3,11a tetrahydro-5H-pyrrolo [2,1-*c*][1,4]benzodiazepin-5-one
2. 7-Methoxy-8-{3-[4-[2-(1,3-dioxo-benz[*de*]isoquinolin-2-yl)ethyl]piperazin-1-yl]propyl}-oxy-(11aS)-1,2,3,11a tetrahydro-5H-pyrrolo [2,1-*c*][1,4]benzodiazepin-5-one
3. 7-Methoxy-8-{4-[4-[2-(1,3-dioxo-benz[*de*]isoquinolin-2-yl)ethyl]piperazin-1-yl]butyl}-oxy-(11aS)-1,2,3,11a tetrahydro-5H-pyrrolo [2,1-*c*][1,4]benzodiazepin-5-one
4. 7-Methoxy-8-{3-[4-[3-(1,3-dioxo-benz[*de*]isoquinolin-2-yl)propyl]piperazin-1-yl]propyl}-oxy-(11aS)-1,2,3,11a tetrahydro-5H-pyrrolo [2,1-*c*][1,4]benzodiazepin-5-one
5. 7-Methoxy-8-{4-[4-[4-(1,3-dioxo-benz[*de*]isoquinolin-2-yl)butyl]piperazin-1-yl]butyl}-oxy-(11aS)-1,2,3,11a tetrahydro-5H-pyrrolo [2,1-*c*][1,4]benzodiazepin-5-one

These new analogues of pyrrolo[2,1-*c*][1,4]benzodiazepine hybrids have shown promising anticancer activity in in selected human cancer cell lines of colon (HT-29, HCT-15), lung (A-549, HOP-62), cervix (SiHa) origin. The molecules synthesized are of immense biological significance with potential sequence selective DNA-binding property. This resulted in design and synthesis of new congeners as illustrated in Scheme-1 which comprises of
1. The ether linkage at C-8 position of DC-81 intermediates with napthalimide moiety.
2. Refluxing the reaction mixture for 24-48h.
3. Synthesis of C-8 linked PBD hybrids.
4. Purification by column chromatography using different solvents like ethyl acetate, hexane, dichloromethane and methanol.

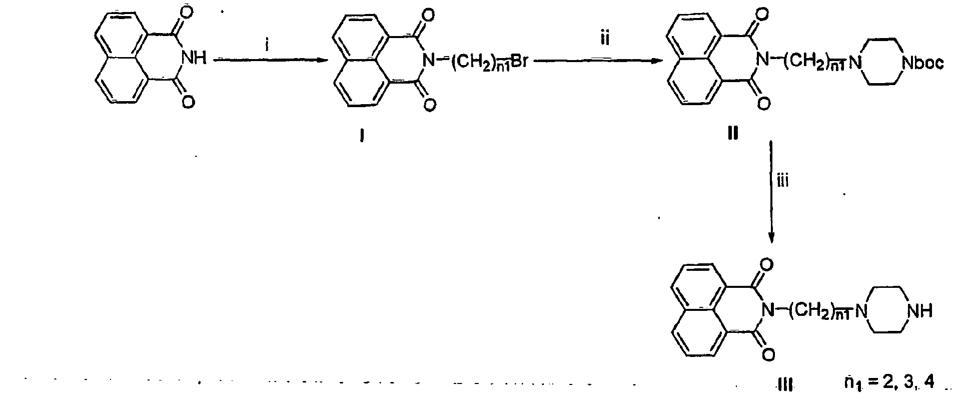

### Reagents and conditions

(i) Dibromo alkanes, K₂CO₃, acetonitrile, reflux, 12h; (ii) N-Boc piperazine, K₂CO₃, acetonitrile, reflux, 8h; (iii) CF₃COOH, CHCl₃, r.t., 12h.

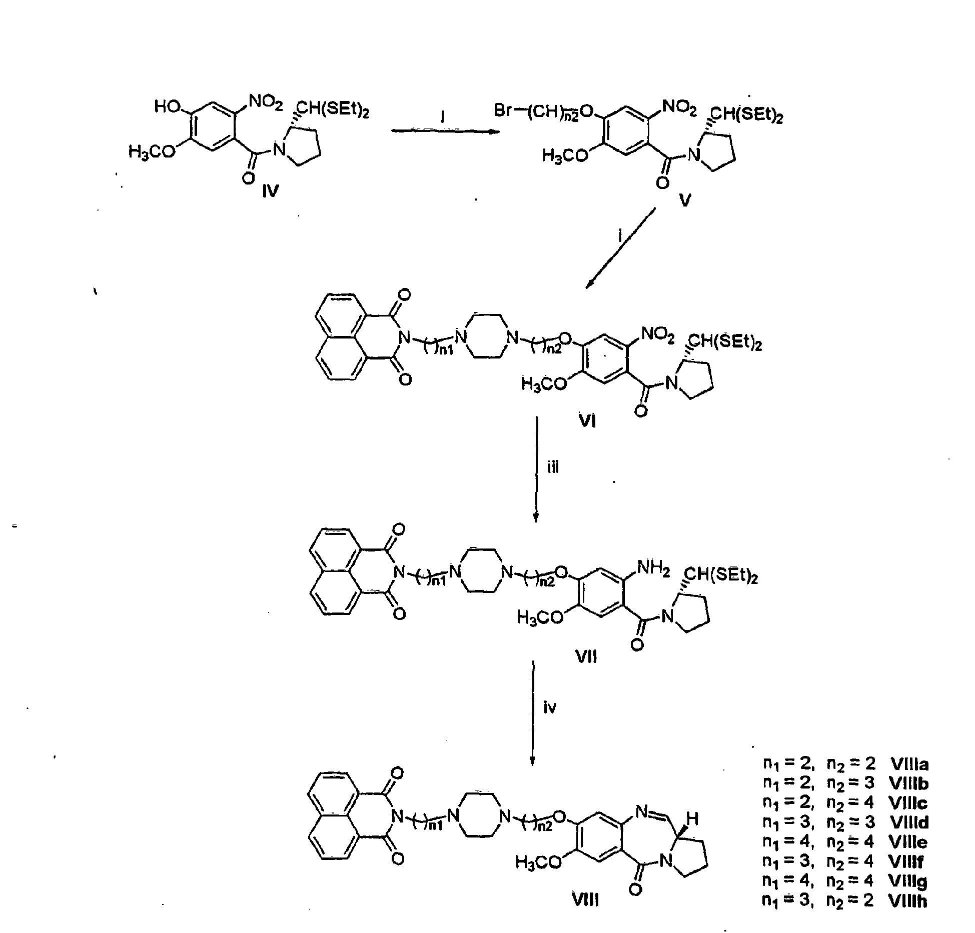

### Reagents and conditions

(i) Dibromo alkanes,K₂CO₃, acetonitrile, reflux, 24 h; (ii) compound I, K₂CO₃, acetonitrile, reflux, 12 h;
(iii) SnCl₂. 2H₂O, methanol, reflux, 5 h; (iv) HgCl₂, CaCO₃, acetonitrile/H₂O, r.t., 12 h

The following examples are given by way of illustration and therefore should not be construed to the present limit of the scope of invention.

**Example 1:** To a solution of (2S)-N-[4-hydroxy-5-methoxy-2-nitrobenzoyl]pyrrolidine-2-carboxaldehyde diethyl thioacetal of formula IV (800 mg, 2 mmol) in acetone were added anhydrous K₂CO₃ (829 mg, 6 mmol) and 1,2 dibromo ethane (940 mg, 5mmol) and the mixture was refluxed for 48h.After completion of reaction K₂CO₃ was removed by filtration and the solvent was evaporated under redused pressure, purification by column chromatography afforded compound V.

¹H NMR (CDCl₃) 1.30-1.45 (m, 6H), 1.70-2.35 (m, 4H), 2.70-2.85 (m, 4H), 3.12-3.30 (m, 2H), 3.70 (t, 2H, J=6.3 Hz), 3.95 (s, 3H), 4.40 (t, 2H, J=6Hz), 4.60-4.75 (m, 1H), 4.82 (d, 1H, J=4.3 Hz), 6.80 (s, 1H), 7.65 (s, 1H).

To a solution of 2S-N-[4-(2-bromo ethoxy)-5-methoxy-2-nitrobenzoyl]pyrrolidine-2-carbaxaldehyde diethyl thioacetal of formula V (507 mg, 1 mmol), piperazine attached naphthalimide (340 mg, 1.1 mmol) in acetone was added anhydrous K₂CO₃ (415 mg, 3 mmol) and the mixture was refluxed for 24h.After completion of reaction K₂CO₃ was removed by filtration and the solvent was evaporated under reduced pressure, purification by column chromatography afforded compound VI.

¹H NMR (CDCl₃, 200 MHz) 1.22-1.40 (m, 6H), 1.70-2.35 (m, 4H), 2.55-2.95 (m, 16H), 3.15-3.32 (m, 2H), 3.92 (s, 3H), 4.15-4.35 (m, 4H), 4.57-4.72 (m, 1H), 4.80 (d, 1H, *J*=4.3 Hz), 6.77 (s, 1H), 7.60-7.80 (m, 3H), 8. 3 0 (t, 2H, *J* = 8 Hz), 8.55 (d, 2H, *J* = 7.6 Hz).

To a solution of 2S-N-{4-[2-[4-[2-(1,3-dioxo-benz[*de*]isoquinolin-2-yl)ethyl]piperazin-1-yl]ethyl]-oxy-5-methoxy-2-nitrobenzoyl}pyrrolidine-2-carbaxaldehyde diethyl thioacetal of formula VI (736 mg, 1 mmol) in methanol was added SnCl₂.2H₂O (1.12 gr, 5 mmol) and the mixture was refluxed until the TLC indicated the completion of reaction. Methanol was evaporated and 10% NaHCO₃ solution was added. Aqueous layer was extracted with ethyl acetate, combined organic phases were dried over Na₂SO₄ and evaporated under vacuum to afford amino thioacetal (VII) and directly used in the next step.

A solution of VII (706 mg, 1 mmol) HgCl₂ (624 mg, 2.3 mmol) and CaCO₃ (250 mg, 2.5 mmol) in CH₃CN-H₂O (4:1) was stirred at room temperature until the TLC indicated complete consumption of the starting material. The reaction mixture was diluted with ethyl acetate and filtered through a celite bed. The organic layer was concentrated, dried and purified by column chromatography to give the compound VIII.

¹H NMR (CDCl₃, 200 MHz) 1.90-2.40 (m, 4H), 2.45-2.92 (m, 12H), 3.55-3.82 (m, 3H), 3.92 (s, 3H), 4.05-4.40 (m, 4H), 6.77 (s, 1H), 7.45 (s, 1H), 7.62 (d, 1H, *J* = 4.39 Hz), 7.76 (t, 2H, *J* = 7.69 Hz), 8,20 (d, 2H, *J*= 8.2 Hz), 8.60 (d, 2H, *J*= 7.32 Hz).

**Example 2:** To a solution of (2S)-N-[4-hydroxy-5-methoxy-2-nitrobenzoyl]pyrrolidine-2-carboxaldehyde diethyl thioacetal of formula IV (800 mg, 2 mmol) in acetone were added anhydrous K₂CO₃ (828 mg, 6 mmol) and 1,3 dibromo propane (1 gr, 5 mmol) and the mixture was refluxed for 48h.After completion of reaction K₂CO₃ was removed by filtration and the solvent was evaporated under reduced pressure, purification by column chromatography afforded compound V.

¹H NMR (CDCl₃, 200 MHz) 1.25-1.40 (m, 6H), 1.72-2.42 (m, 6H), 2.70-2.8 (m, 4H), 3.15-3.30 (m, 2H), 3.60 (t, 2H, *J* = 6.20 Hz), 3.95 (s, 3H), 4.20 (t, 2H, *J =* 4.96 Hz), 4.60-4.75 (m, 1H), 4.82 (d, 1H, *J*=4.33 Hz), 6.78 (s, 1H), 7.68 (s, 1H).

To a solution of 2S-N-[4-(3-bromo propoxy)-5-methoxy-2-nitrobenzoyl]pyrrolidine-2-carbaxaldehyde diethyl thioacetal of formula V (521 mg, 1 mmol), piperazine attached naphthalimide (340 mg, 1.1 mmol) in acetone was added anhydrous K₂CO₃ (415 mg, 3 mmol) and the mixture was refluxed for 24h.After completion of reaction K₂CO₃ was removed by filtration and the solvent was evaporated under reduced pressure, purification by column chromatography afforded compound VI.

¹H NMR (CDCl₃, 200 MHz) 1.30-1.42 (m, 6H), 1.70-2.30 (m, 6H), 2.40-2.82 (m, 16H), 3.15-3.30 (m, 2H), 3.92 (s, 3H), 4.15 (m, 2H), 4.30 (m , 2H), 4.60-4.70 (m, 1H), 4.82 (d, 1H, *J*=4.25 Hz), 6.75 (s, 1H), 7.65 (s, 1H), 7.75 (t, 2H, *J*=7.4 Hz), 8.2 (d, 2H, *J*= 8 Hz), 8.6 (d, 2H, *J*= 7.6 Hz).

To a solution of 2S-N-{4-[3-[4-[2-(1,3-dioxo-benz[*de*]isoquinolin-2-yl)ethyl]piperazin-1-yl]propyl]-oxy-5-methoxy-2-nitrobenzoyl}pyrrolidine-2-carbaxaldehyde diethyl thioacetal of formula VI (750 mg, 1 mmol) .in methanol was added SnCl₂.2H₂O (1.12 gr, 5 mmol) and the mixture was refluxed until the TLC indicated the completion of reaction. Methanol was evaporated and 10% NaHCO₃ solution was added. Aqueous layer was extracted with ethyl acetate. Combined organic phases were dried over Na₂SO₄ and evaporated under vacuum to afford amino thioacetal (VII) and directly used in the next step.

A solution of VII (720 mg, 1 mmol) HgCl₂ (624 mg, 2.3 mmol) and CaCO₃ (250 mg, 2.5 mmol) in CH₃CN-H₂O (4:1) was stirred at room temperature until the TLC indicated complete consumption of the starting material. The reaction mixture was diluted with ethyl acetate and filtered through a celite bed. The organic layer was concentrated, dried and purified by column chromatography to give the compound VIII.

¹H NMR (CDCl₃, 200 MHz) 1.60-2.16 (m, 6H), 2.25-2.80 (m, 12H), 3.50-3.82 (m, 3H), 3.95 (s, 3H), 4.05-4.40 (m, 4H), 6.80 (s, 1H), 7.45 (s, 1H), 7.62 (d, 1H, *J*= 3.33 Hz), 7.79 (t, 2H, *J* = 7.32 Hz), 8.20 (d, 2H, *J=* 8.05 Hz), 8.60 (d, 2H, *J*= 7.32 Hz).

**Example 3:** To a solution of (2S)-N-[4-hydroxy-5-methoxy-2-nitrobenzoyl]pyrrolidine-2-carboxaldehyde diethyl thioacetal of formula (800 mg, 2 mmol) in acetone were added anhydrous K₂CO₃ (829 mg, 6 mmol) and 1,4 dibromo butane (1.07 gr, 5 mmol) and the mixture was refluxed for 48h.After completion of reaction K₂CO₃ was removed by filtration and the solvent was evaporated under redused pressure, purification by column chromatography afforded compound V.

¹H NMR (CDCl₃, 300 MHz) 1.30-1.40 (m, 6H), 1.75-2.40 (m, 8H), 2.70-2.85 (m, 4H), 3.15-3.30 (m, 2H), 3.50 (t, 2H, *J* = 6.25 Hz), 3.95 (s, 3H), 4.10 (m, 2H), 4.60-4.70 (m, 1H), 4.82 (d, 1H, *J*=4.3 Hz), 6.75 (s, 1H), 7.62 (s, 1H).

To a solution of 2S-N-[4-(4-bromo butoxy)-5-methoxy-2-nitrobenzoyl]pyrrolidine-2-carbaxaldehyde diethyl thioacetal of formula V (535 mg, 1 mmol), piperazine attached naphthalimide (340 mg, 1.1 mmol) in acetone was added anhydrous K₂CO₃ (415 mg, 3 mmol) and the mixture was refluxed for 24h.After completion of reaction K₂CO₃ was removed by filtration and the solvent was evaporated under redused pressure, purification by column chromatography afforded compound VI.

¹H NMR (CDCl₃, 200 MHz) 1.25-1.40 (m, 6H), 1.60-2.15 (m, 8H), 2.35-2.85 (m, 16H), 3.15-3.30 (m, 2H), 3.92 (s, 3H), 4.12 (m, 1H), 4.30 (m, 2H), 4.60-4.72 (m, 1H), 4.80 (d, 1H, *J*=4.23 Hz), 6.75 (s, 1H), 7.60 (s, 1H), 7.75 (t, 2H, *J*=7.45 Hz), 8.2 (d, 2H, *J*= 8.25 Hz), 8.56 (d, 2H, *J*= 7.62 Hz).

To a solution of 2S-N-{4-[4-[2-(1,3-dioxo-benz[*de*]isoquinolin-2-yl)ethyl]piperazin-1-yl]butyl]-oxy-5-methoxy-2-nitrobenzoyl}pyrrolidine-2-carbaxaldehyde diethyl thioacetal (764 mg, 1 mmol) of formula VI .in methanol was added SnCl₂.2H₂O (1.12 gr, 5 mmol) and the mixture was refluxed until the TLC indicated the completion of reaction. The methanol was evaporated and 10% NaHCO₃ solution was added. The aqueous layer was extracted with ethyl acetate, the combined organic phases were dried over Na₂SO₄ and evaporated under vacuum to afford the amino thioacetal (VII) and directly used in the next step.

A solution of VII (734 mg, 1 mmol) HgCl₂ (624 mg, 2.3 mmol) and CaCO₃ (250 mg, 2.5 mmol) in CH₃CN-H₂O (4:1) was stirred at room temperature until the TLC indicated complete consumption of the starting material. The reaction mixture was diluted with ethyl acetate and filtered through a celite bed. The organic layer was concentrated, dried and purified by column chromatography to give the compound VIII.

¹H NMR (CDCl₃, 200 MHz) 1.56-2.15 (m, 8H), 2.25-2.80 (m, 16H), 3.45-3.82 (m, 3H), 3.92 (s, 3H), 4.0-4.15 (m, 2H), 4.22-4.37 (t, 2H), 6.70 (s, 1H), 7.42 (s, 1H), 7.60 (d, 1H, *J* = 4.25 Hz), 7.72 (t, 2H, *J* = 7.4 Hz), 8.16 (d, 2H, *J*= 8.1 Hz), 8.56 (d, 2H, *J* = 7.42 Hz).

**Example 4:** To a solution of (25)-N-[4-hydroxy-5-methoxy-2-nitrobenzoyl]pyrrolidine-2-carboxaldehyde diethyl thioacetal of formula IV (800 mg, 2 mmol) in acetone were added anhydrous K₂CO₃ (829 mg, 6 mmol) and 1,3 dibromo propane (1 gr, 5mmol) and the mixture was refluxed for 48h.After completion of reaction K₂CO₃ was removed by filtration and the solvent was evaporated under redused pressure, purification by column chromatography afforded compound V.

¹H NMR (CDCl₃, 200 MHz) 1.25-1.40 (m, 6H), 1.72-2.42 (m, 6H), 2.70-2.8 (m, 4H), 3.15-3.30 (m, 2H), 3.60 (t, 2H, *J* = 6.20 Hz), 3.95 (s, 3H), 4.20 (t, 2H, *J*= 4.96 Hz), 4.60-4.75 (m, 1H), 4.82 (d, 1H, *J*=4.33 Hz), 6.78 (s, 1H), 7.68 (s, 1H).

To a solution of 2S-N-[4-(3-bromo propoxy)-5-methoxy-2-nitrobenzoyl]pyrrolidine-2-carbaxaldehyde diethyl thioacetal of formula V (521 mg, 1 mmol), piperazine attached naphthalimide (324 mg, 1 mmol) in acetone were added anhydrous K₂CO₃ (415 mg, 3 mmol) and the mixture was refluxed for 24h.After completion of reaction K₂CO₃ was removed by filtration and the solvent was evaporated under reduced pressure, purification by column chromatography afforded compound VI.

¹H NMR (CDCl₃, 200 MHz) 1.25-1.42 (m, 6H), 1.70-2.40 (m, 8H), 2.60-3.30 (m, 18H), 3.92 (s, 3H), 4.05 (m, 4H), 4.70-4.80 (m, 1H), 4.82 (d, 1H, *J*=4.25 Hz), 6.77 (s, 1H), 7.60 (s, 1H), 7.75 (t, 2H, *J*=7.3 5 Hz), 8.18 (d, 2H, *J*= 8 Hz), 8.55 (d, 2H, *J*= 7.55 Hz).

To a solution of 2S-N-{4-[3-[3-[4-[3-(1,3-dioxo-benz[*de*]isoquinolin-2-yl)propyl]piperazin-1-yl]propyl]-oxy-5-methoxy-2-nitrobenzoyl}pyrrolidine-2-carbaxaldehyde diethyl thioacetal of formula VI (764 mg, 1 mmol) .in methanol was added SnCl₂.2H₂O (1.12 gr, 5 mmol) and the mixture was refluxed until the TLC indicated the completion of reaction. The methanol was evaporated and 10% NaHCO₃ solution was added. The aqueous layer was extracted with ethyl acetate, the combined organic phases were dried over Na₂SO₄ and evaporated under vacuum to afford the amino thioacetal (VII) and directly used in the next step.

A solution of VII (734 mg, 1 mmol) HgCl₂ (624 mg, 2.3 mmol) and CaCO₃ (250 mg, 2.5 mmol) in CH₃CN-H₂O (4:1) was stirred at room temperature until the TLC indicated complete consumption of the starting material. The reaction mixture was diluted with ethyl acetate and filtered through a celite bed. The organic layer was concentrated, dried and purified by column chromatography to give the compound VIII.

¹H NMR (CDCl₃, 200 MHz) 1.75-2.18 (m, 8H), 2.22-2.80 (m, 16H), 3.45-4.30 (m, 10H), 6.75 (s, 1H), 7.45 (s, 1H), 7.60 (d, 1H, *J =* 4.2 Hz), 7.72 (t, 2H, *J* = 7.4 Hz), 8.20 (d, 2H, *J =* 8.1 Hz), 8.58 (d, 2H, *J*= 7.35 Hz).

**Example 5:** To a solution of (2S)-N-[4-hydroxy-5-methoxy-2-nitrobenzoyl]pyrrolidine-2-carboxaldehyde diethyl thioacetal of formula IV (800 mg, 2 mmol) in acetone were added anhydrous K₂CO₃ (829 mg, 6 mmol) and 1,4 dibromo butane (1 gr, 5 mmol) and the mixture was refluxed for 48h.After completion of reaction K₂CO₃ was removed by filtration and the solvent was evaporated under redused pressure, purification by column chromatography afforded compound V.

¹H NMR (CDCl₃, 300 MHz) 1.30-1.40 (m, 6H), 1.75-2.40 (m, 8H), 2.70-2.85 (m, 4H), 3.15-3.30 (m, 2H), 3.50 (t, 2H, *J* = 6.25 Hz), 3.95 (s, 3H), 4.10 (m, 2H), 4.60-4.70 (m, 1H), 4.82 (d, 1H, *J*=4.3 Hz), 6.75 (s, 1H), 7.62 (s, 1H).

To a solution of 2S-N-[4-(4-bromo butoxy)-5-methoxy-2-nitrobenzoyl]pyrrolidine-2-carbaxaldehyde diethyl thioacetal of formula V (538mg, 1 mmol), piperazine attached naphthalimide (337 mg, 1 mmol) in acetone were added anhydrous K₂CO₃ (415 mg, 3 mmol) and the mixture was refluxed for 24h.After completion of reaction K₂CO₃ was removed by filtration and the solvent was evaporated under reduced pressure, purification by column chromatography afforded compound VI.

¹H NMR (CDCl₃, 200 MHz) 1.60-2.33 (m, 12H), 2.52-3.0 (m, 16H), 3.12-3.30 (m, 2H), 3.95 (s, 1H), 4.02-4.25 (m, 4H), 4.60-4.72 (m, 1H), 4.80 (d, 1H, *J*=4.3 Hz), 6.75 (s, 1H), 7.60 (s, 1H), 7.75 (t, 2H, *J*=7.45 Hz), 8.18 (d, 2H, *J*= 8.2 Hz), 8.56 (d, 2H, *J*= 7.6 Hz).

To a solution of 2S-N-{4-[4-[4-[4-(1,3-dioxo-benz[*de*]isoquinolin-2-yl)butyl]piperazin-1-yl]butyl]-oxy-5-methoxy-2-nitrobenzoyl}pyrrolidine-2-carbaxaldehyde diethyl thioacetal (792 mg, 1 mmol) of formula VI .in methanol was added SnCl_{2.}2H₂O (1.12 gr, 5 mmol) and the mixture was refluxed until the TLC indicated the completion of reaction. The methanol was evaporated and 10% NaHCO₃ solution was added. The aqueous layer was extracted with ethyl acetate, the combined organic phases were dried over Na₂SO₄ and evaporated under vacuum to afford amino thioacetal (VII) and directly used in the next step.

A solution of VII (762 mg, 1 mmol) HgCl₂ (624 mg, 2.3 mmol) and CaCO₃ (250 mg, 2.5 mmol) in CH₃CN-H₂O (4:1) was stirred at room temperature until the TLC indicated complete consumption of the starting material. The reaction mixture was diluted with ethyl acetate and filtered through a celite bed. The organic layer was concentrated, dried and purified by column chromatography to give the compound VIII.

¹H NMR (CDCl₃, 300 MHz) 1.50-2.10 (m, 12H), 2.25-2.80 (m, 16H), 3.45-4.30 (m, 10H), 6.75 (s, 1H), 7.45 (s, 1H), 7.62 (d, 1H, *J* = 4.2 Hz), 7.75 (t, 2H, *J* = 7.3 Hz), 8.20 (d, 2H, *J* = 8.1 Hz), 8.56 (d, 2H, *J*= 7.40 Hz).

### Biological activity:

*In vitro* cytotoxicity against human cancer cell lines: The human cancer cell lines procured from National Cancer Institute, Frederick, U.S.A or National Center for Cell Science; Pune, India. were used in present study. Cells were grown in tissue culture flasks in complete growth medium (RPMI-1640 medium with 2 mM glutamine, 100 µg/ml streptomycin, pH 7.4, sterilized by filtration and supplemented with 10% fetal calf serum and 100 units/ml penicillin before use) at 37°C in an atmosphere of 5% CO₂ and 90% relative humidity in a carbon dioxide incubator. The cells at subconfluent stage were harvested from the flask by treatment with trypsin (0.5% in PBS containing 0.02% EDTA) for determination of cytotoxicity. Cells with viability of more than 98% as determined by trypan blue exclusion were used for assay. The cell suspension of the required cell density were prepared in complete growth medium with gentamycin (50µg/ml) for determination of cytotoxicity.

A stock solutions of (2 X 10⁻² M) of test material were prepared in DMSO . The stock solutions were serially diluted with complete growth medium containing 50µg/ml of gentamycin to obtain working test solutions of required concentrations.

*In vitro* cytotoxicity against human cancer cell lines was determined (Monks, A., Scudiero, D., Skehan, P, Shoemaker R, Paull, K., Vistica, D., Hose, C., Langley, J., Cronise, P., Vaigro-Wolff, A., Gray-Goodrich, M.; Campbell,H., Mayo, J and Boyd M. J. Natl. Cancer Inst.,1991,83, 757-766) using 96-well tissue culture plates. The 100 µl of cell suspension was added to each well of the 96-well tissue culture plate. The cells were incubated for 24 hours. Test materials in complete growth medium (100µl) were added after 24 hours incubation to the wells containing cell suspension. The plates were further incubated for 48 hours (at 37°C in an atmosphere of 5% and 90% relative humidity in a carbon dioxide incubator) after addition of test material and then the cell growth was stopped by gently layering trichloroacetic acid (TCA, 50µl, 50%) on top of the medium in all the wells. The plates were incubated at 4°C for one hour to fix the cells attached to the bottom of the wells. The liquid of all the wells was gently pipetted out and discarded. The plates were washed five times with distilled water to remove TCA, growth medium low molecular weight metabolites, serum proteins etc and air-dried. Cell growth was measured by staining with sulforhodamine B dye (Skehan *et al.,* 1990). The adsorbed dye was dissolved in Tris-Buffer (100 ml, 0.01M, pH 10.4) and plates were gently stirred for 5 minutes on a mechanical stirrer. The optical density was recorded on ELISA reader at 540 nm.

The cell growth was calculated by subtracting mean OD value of respective blank from the mean OD value of experimental set. Percent growth in presence of test material was calculated considering the growth in absence of any test material as 100% and in turn percent growth inhibition in presence of test material will be calculated.

### Cytotoxicity:

Compounds were evaluated for the primary anticancer activity. The cytotoxicity data for some representative compounds has shown in Table 1.

**Table 1. The percentage growth inhibition data for napthalimide-PBD hybrids**

| Compd | Cancer cell lines | | | | | | | | | | | | | | |
|---|---|---|---|---|---|---|---|---|---|---|---|---|---|---|---|
| | HT-29 | | | HCT-15 | | | A-549 | | | HOP-62 | | | SiHa | | |
| (mol/L) | 10⁻⁴ | 10⁻⁵ | 10⁻⁶ | 10⁻⁴ | 10⁻⁵ | 10⁻⁶ | 10⁻⁴ | 10⁻⁵ | 10⁻⁶ | 10⁻⁴ | 10⁻⁵ | 10⁻⁶ | 10⁻⁴ | 10⁻⁵ | 10⁻⁶ |
| **VIIIb** | 88 | 85 | 91 | a | 59 | 58 | 88 | a | a | a | 86 | 72 | a | 44 | 54 |
| **VIIIc** | 94 | 86 | 80 | a | 69 | 60 | 95 | a | a | a | 93 | 86 | 56 | 39 | a |
| **VIIId** | 95 | 62 | 63 | a | a | a | 95 | 30 | 51 | 78 | a | a | 85 | 65 | 31 |
| **VIIIe** | 92 | 78 | 67 | a | 62 | 59 | 93 | a | a | a | 93 | 80 | 42 | 40 | 44 |

| | | | | | | | | | | | | | | | |
|---|---|---|---|---|---|---|---|---|---|---|---|---|---|---|---|
| a:not tested | | | | | | | | | | | | | | | |

**Table 2. DNA Thermal Denaturation Studies:**

| | Induced □*Tₘ* °C after incubation at 37 °C | |
|---|---|---|
| Compound | 0 h | 18 h |
| **VIIIa** | 21.9 | 22.7 |
| **VIIIb** | 25.8 | 26.7 |
| **VIIIc** | 23.4 | 24.2 |
| **VIIId** | 13.1 | 14.3 |
| **VIIIe** | 20.9 | 21.7 |
| DC-81 | 0.3 | 0.7 |

For CT-DNA alone at pH 7.00 ± 0.01, *Tₘ* = 69.8 °C ± 0.01 (mean value from 10 separate determinations), all Δ*Tₘ* values are ± 0.1 - 0.2 °C. For a 1:5 molar ratio of [PBD]/[DNA], where CT-DNA concentration = 100 µM and ligand concentration = 20 µM in aqueous sodium phosphate buffer [10 mM sodium phosphate + 1 mM EDTA, pH 7.00 ± 0.01].

## Claims

1. A novel pyrrolo[2,1-*c*][1,4]benzodiazepine of formula VIII where n₁ = 2, 3, 4, n₂ = 2, 3, 4.

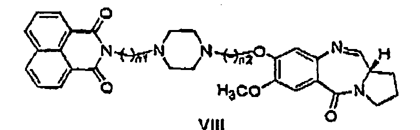

2. A compound of claim 1 which is 7-methoxy-8-{2-[4-[2-(1,3-dioxo-benz[*de*]isoquinolin-2-yl)alkyl]piperazin-1-yl]alkyl}-oxy-(11aS)-1,2,3,11a tetrahydro-5H-pyrrolo [2,1-*c*][1,4]benzodiazepin-5-one of formula VIII where alkyl = ethyl, propyl, butyl.

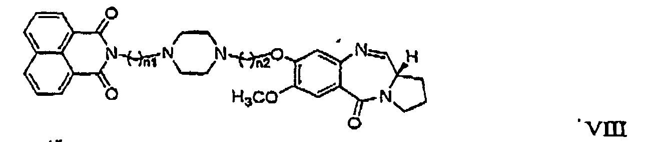

3. A compound of claim 1 which is 7-methoxy-8-{2-[4-[2-(1,3-dioxo-benz[*de*]isoquinolin-2-yl)ethyl]piperazin-1-yl]ethyl}-oxy-(11aS)-1,2,3,11a tetrahydro-5H-pyrrolo [2,1-*c*][1,4]benzodiazepin-5-one of the structural formula shown below.

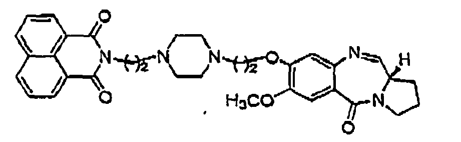

4. A compound of claim 1 which is 7-methoxy-8-{3-[4-[2-(1,3-dioxo-benz[*de*]isoquinolin-2-yl)ethyl]piperazin-1-yl]propyl}-oxy-(11aS)-1,2,3,11a tetrahydro-5H-pyrrolo [2,1-*c*][1,4]benzodiazepin-5-one of the structural formula shown below.

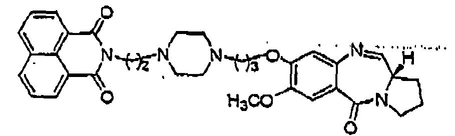

5. A compound of claim 1 which is 7-ethoxy-8-{4-[4-[2-(1,3-dioxo-benz[*de*]isoquiuolin-2-yl)ethyl]piperazin-1-yl]butyl}-oxy-(11aS)-1,2,3,11a tetrahydro-5H-pyrrolo [2,1-*c*][1,4]benzodiazepin-5-one of the structural formula shown below.

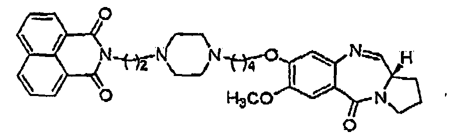

6. A compound of claim 1 which is 7-methoxy-8-{3-[4-(3-(1,3-dioxo-benz[*de*]isoquiaolin-2-yl)propyl]piperazin-1-yl]propyl}-oxy-(11aS)-1,2,3,11a tetrahydro-5H-pyrrolo [2,1-*c*][1,4]benzodiazepin-5-one of the structural formula shown below.

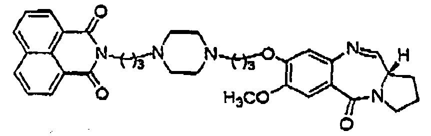

7. A compound of claim 1 which is 7-methoxy-8-{4-[4-[4-(1,3-dioxo-benz[*de*]isoquinolin-2-yl)butyl]piporazin-1-yl]butyl}-oxy-(11aS)-1,2,3,11a tetrahydro-5H-pyrrolo [2,1-*c*][1,4]benzodiazepin-5-one of the structural formula shown below.

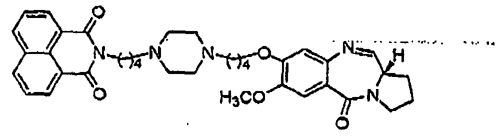

8. A compound of claim 1 which is 7-methoxy-8-{4-[4-[2-(1,3-dioxo-benz[*de*]isoqpinolin-2-yl)propyl]piperazin-1-yl]butyl}-oxy-(11aS)-1,2,3,11a tetrahydro-5H-pyrrolo [2,1-*c*][1,4]benzodiazepin-5-one of the structural formula shown below.

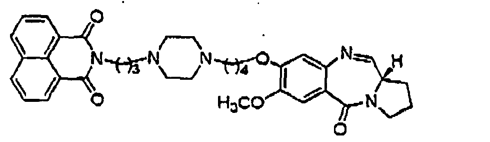

9. A compound of claim 1 which is 7-methoxy-8-{3-[4-[2-(1,3-dioxo-benz[*de*]isoquinolin-2-yl)butyl]piperazin-1-yl]propyl}-oxy-(11aS)-1,2,3,11a tetrahydro-5H-pyrrolo [2,1-*c*][1,4]benzodiazepin-5-one of the structural formula shown below.

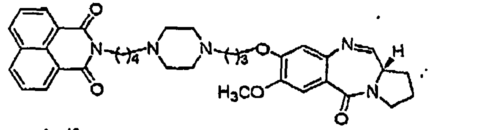

10. A compound of claim 1 which is 7-methoxy-8-{3-[4-[3-(1,3-dioxo-benz[*de*]isoquinolin-2-yl)propyl]piperazin-1-yl]ethyl}-oxy-(11aS)-1,2,3,11a tetrahydro-5H-pyrrolo [2,1-*c*][1,4]benzodiazepin-5-one of the structural formula shown below.

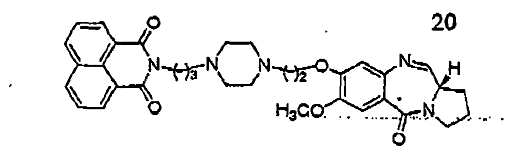

11. A process for the preparation of pyrolo[2,1-*c*][1,4]benzodiazopine of ormula VIII 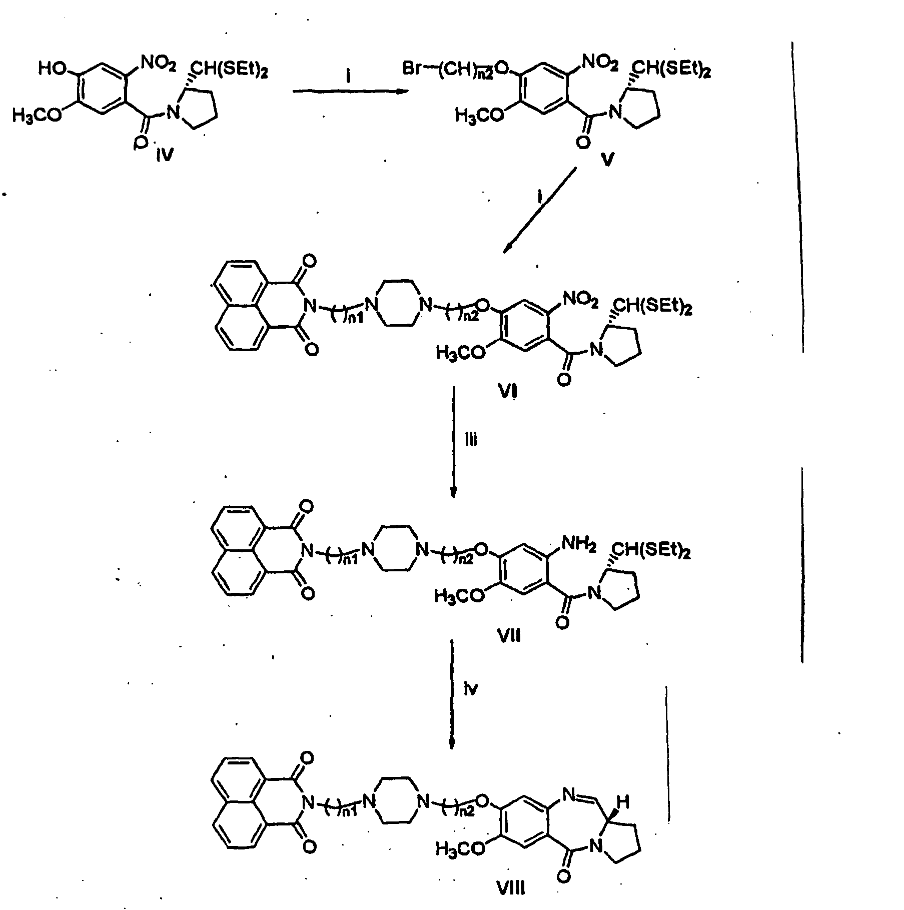 where n1 = 2, 3, 4, n2 = 2, 3, 4, which comprises of reacting (2S)-N-[4-hydroxy-5-methoxy-2-nitrobenzoyl]pyrroloidine-2-carboxaldehyde diethyl thioacetal of formula IV with dibromoalkanes in an aprotic water miscible solvent in the presence of a mild inorganic base at refluxing temperature for a period of 48h, isolating 2S-N-[4-(n-bromo alkoxy)-5-methoxy-2-nitrobenzoyl]pyrrolidine-2-carbaxaldehyde diethyl thioacetyl of formula V, reacting the compound of formula V with piperazine attached naphthalimide in presence of mild inorganic bases isolating compound of formula VI. reducing it with SnCl₂.2H₂O in presence of organic solvent at a reflux temperature, reacting the above amino compound of formula VII with a deprotecting agent to obtain the pyrrol[2,1-*c*][1,4]benzodiazepine of formula VIII wherein n is as stated above.

12. A compound according to any of claims 1 to 10 or a pharmaceutically acceptable derivative or salt thereof for use in the treatment of cancer.

13. The use of a compound according to any of claims 1 to 10 or a pharmaceutically acceptable derivative or salt thereof in the manufacture of a medicament for the treatment of cancer.

14. A pharmaceutical composition comprising a pharmaceutically effective amount of a compound according to any of claims 1 to 10 or a pharmaceutically acceptable derivative or salt thereof and a pharmaceutically acceptable excipient.

## Patentansprüche

1. Neuartiges Pyrrolo[2,1-*c*][1,4]benzodiazepin der Formel VIII, wobei n₁ = 2, 3, 4 und n₂ = 2, 3, 4 ist.

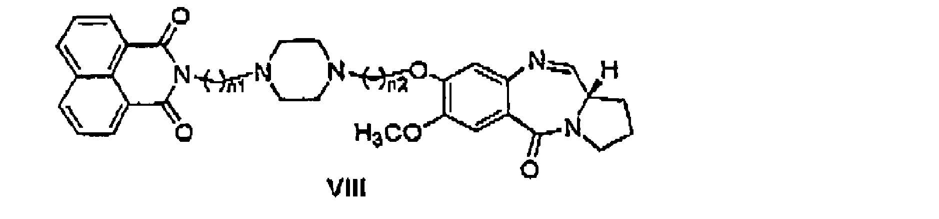

2. Verbindung nach Anspruch 1, die 7-Methoxy-8-{2-[4-[2-(1,3-dioxo-benz[*de*]isochinolin-2-yl)alkyl]piperazin-1-yl]alkyl}-oxy-(11 aS)-1,2,3,11 a-tetra-hydro-5H-pyrrolo[2,1-*c*][1,4]benzodiazepin-5-on der Formel VIII ist, wobei Alkyl = Ethyl, Propyl, Butyl ist.

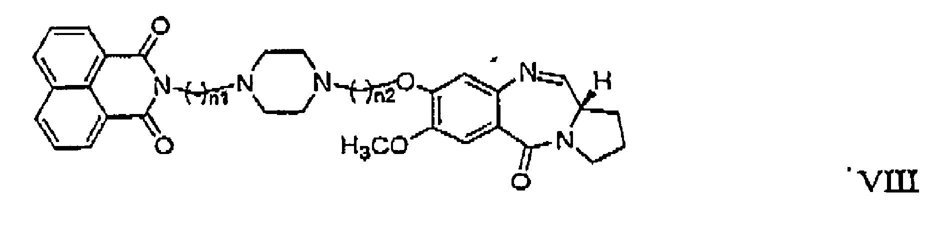

3. Verbindung nach Anspruch 1, die 7-Methoxy-8-{2-[4-[2-(1,3-dioxobenz[*de*]isochinolin-2-yl)ethyl]piperazin-1-yl]ethyl}-oxy-(11aS)-1,2,3,11a-tetrahydro-5H-pyrrolo[2,1-*c*][1,4]benzodiazepin-5-on der unten gezeigten Strukturformel ist.

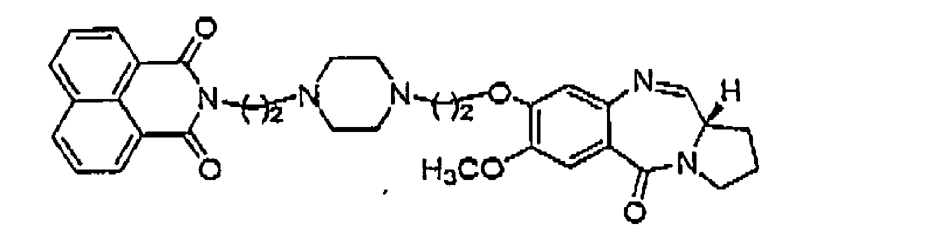

4. Verbindung nach Anspruch 1, die 7-Methoxy-8-{3-[4-[2-(1,3-dioxobenz[*de*]isochinolin-2-yl)ethyl]piperazin-1-yl]propyl}-oxy-(11aS)-1,2,3,11a-tetrahydro-5H-pyrrolo[2,1-*c*][1,4]benzodiazepin-5-on der unten gezeigten Strukturformel ist.

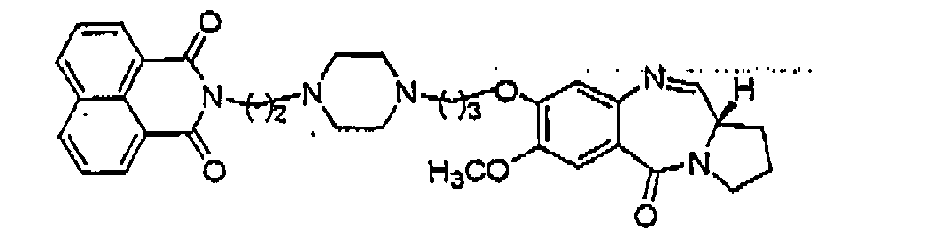

5. Verbindung nach Anspruch 1, die 7-Methoxy-8-{4-[4-[2-(1,3-dioxobenz[*de*]isochinolin-2-yl)ethyl]piperazin-1-yl]butyl}-oxy-(11aS)-1,2,3,11a-tetrahydro-5H-pyrrolo[2,1-*c*][1,4]benzodiazepin-5-on der unten gezeigten Strukturformel ist.

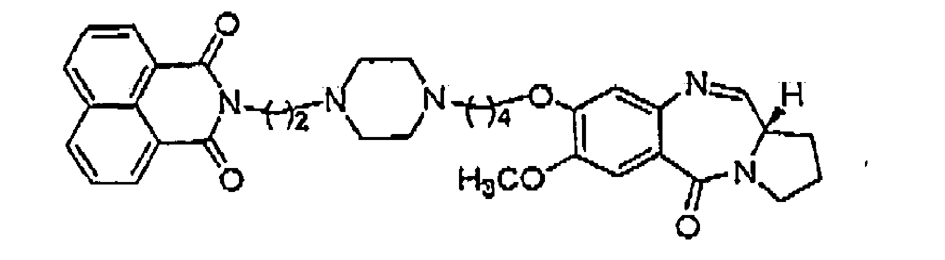

6. Verbindung nach Anspruch 1, die 7-Methoxy-8-{3-[4-[3-(1,3-dioxobenz[*de*]isochinolin-2-yl)propyl]piperazin-1-yl]propyl}-oxy-(11aS)-1,2,3,11a-tetrahydro-5H-pyrrolo[2,1-*c*][1,4]benzodiazepin-5-on der unten gezeigten Strukturformel ist.

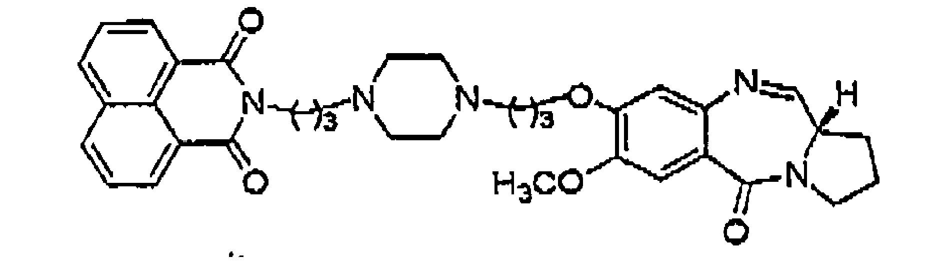

7. Verbindung nach Anspruch 1, die 7-Methoxy-8-{4-[4-[4-(1,3-dioxobenz[*de*]isochinolin-2-yl)butyl]piperazin-1-yl]butyl}-oxy-(11aS)-1,2,3,11a-tetrahydro-5H-pyrrolo[2,1-*c*][1,4]benzodiazepin-5-on der unten gezeigten Strukturformel ist.

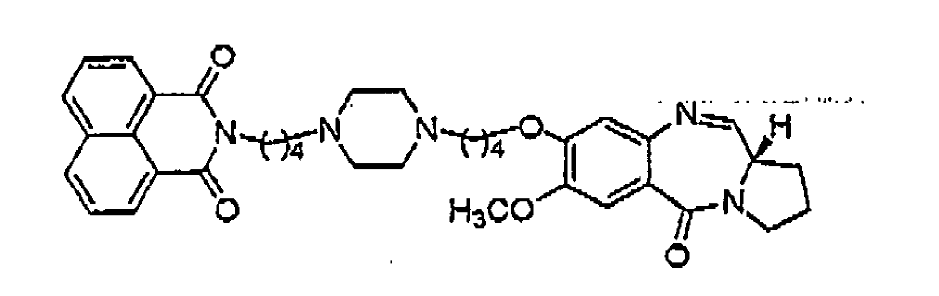

8. Verbindung nach Anspruch 1, die 7-Methoxy-8-{4-[4-2-(1,3-dioxobenz[*de*]isochinolin-2-yl)propyl]piperazin-1-yl]buty1}-oxy-(11aS)-1,2,3,11a-tetrahydro-5H-pyrrolo[2,1-*c*][1,4]benzodiazepin-5-on der unten gezeigten Strukturformel ist.

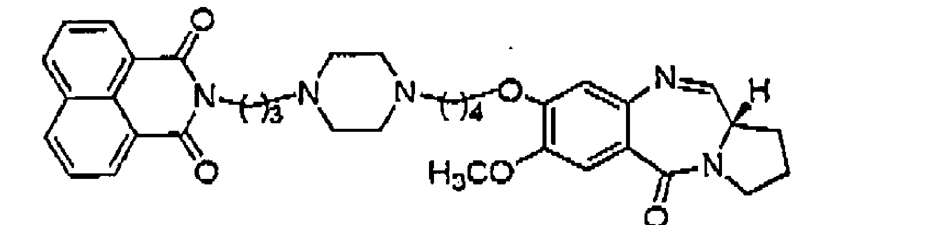

9. Verbindung nach Anspruch 1, die 7-Methoxy-8-{3-[4-[2-(1,3-dioxobenz[*de*]isochinolin-2-yl)butyl]piperazin-1-yl]propyl}-oxy-(11aS)-1,2,3,11a-tetrahydro-5H-pyrrolo[2,1-*c*][1,4]benzodiazepin-5-on der unten gezeigten Strukturformel ist.

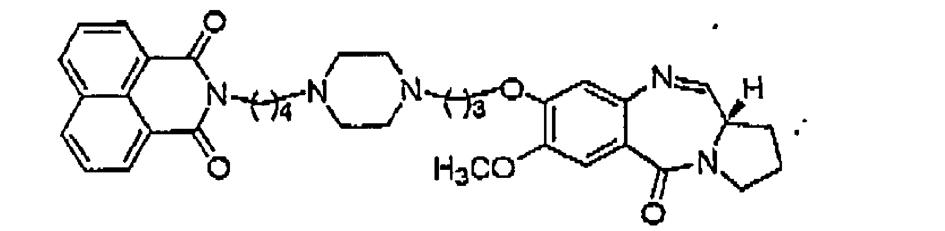

10. Verbindung nach Anspruch 1, die 7-Methoxy-8-{3-[4-[3-(1,3-dioxobenz[*de*]isochinolin-2-yl)propyl]piperazin-1-yl]ethyl}-oxy-(11aS)-1,2,3,11a-tetrahydro-5H-pyrrolo[2,1-*c*][1,4]benzodiazepin-5-on der unten gezeigten Strukturformel ist.

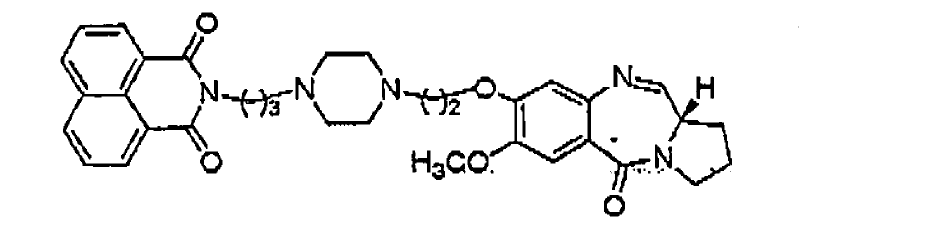

11. Verfahren zur Herstellung von Pyrrolo[2,1-*c*][1,4]benzodiazopinen der Formel VIII 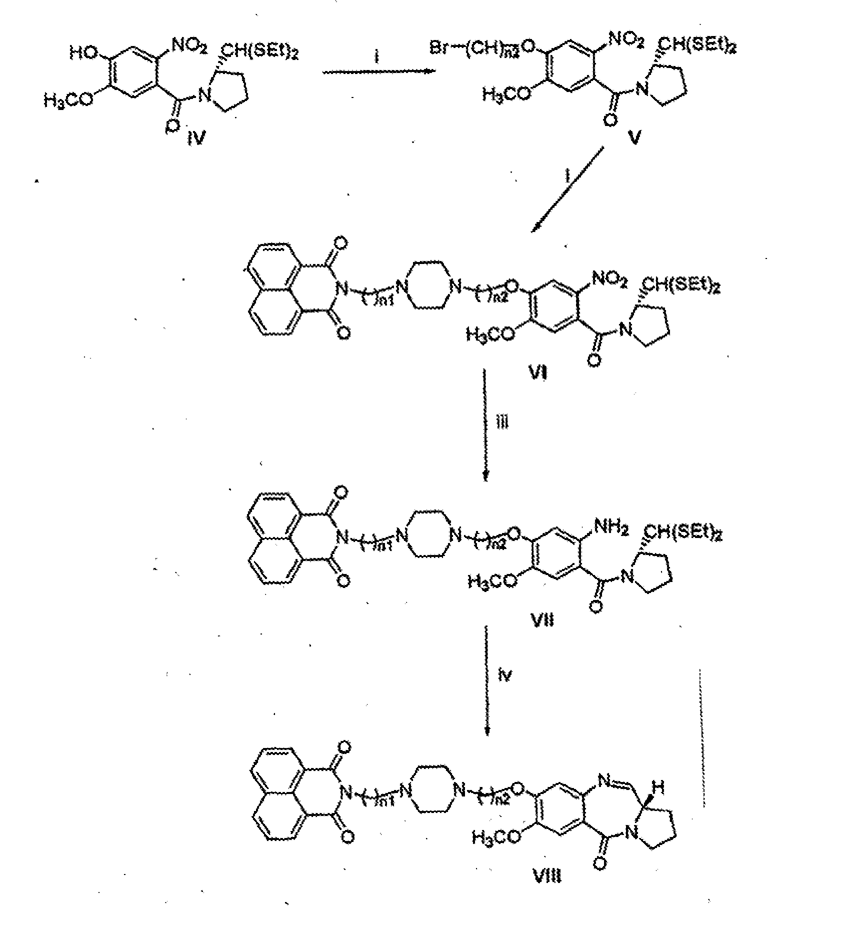 mit n1 = 2, 3, 4 und n2 = 2, 3, 4, umfassend das Reagieren von (2S)-N-[4-hydroxy-5-methoxy-2-nitrobenzoyl]pyrroloidin-2-carboxaldehyddiethylthioacetal der Formel IV mit Dibromalkanen in einem aprotischen, mit Wasser mischbaren Lösungsmittel in Anwesenheit einer milden anorganischen Base bei Rückflusstemperatur für eine Zeitdauer von 48 h, das Isolieren von (2S)-N-[4-(n-Bromalkoxy)-5-methoxy-2-nitrobenzoyl]pyrrolidin-2-carbaxyldehyddiethylthioacetyl der Formel V, das Reagieren der Verbindung der Formel V mit an Piperazin angehängtem Napththalimid in Anwesenheit einer milde organische Basen isolierenden Verbindung der Formel VI, das Reduzieren derselben mit SnCl₂.2H₂O in Anwesenheit eines organischen Lösungsmittels bei einer Rückflusstemperatur, das Reagieren der obigen Aminoverbindung der Formel VII mit einem Entschützungsmittel, um das Pyrrolo[2,1-*c*][1,4]benzodiazepin der Formel VII zu erhalten, wobei n die oben angegebene Bedeutung besitzt.

12. Verbindung nach einem der Ansprüche 1 bis 10 oder pharmazeutisch annehmbares Derivat oder Salz derselben zur Verwendung bei der Behandlung von Krebs.

13. Verwendung einer Verbindung nach einem der Ansprüche 1 bis 10 oder eines pharmazeutisch annehmbaren Derivats oder Salzes derselben bei der Herstellung eines Medikaments für die Behandlung von Krebs.

14. Pharmazeutische Zusammensetzung umfassend eine pharmazeutisch wirksame Menge einer Verbindung nach einem der Ansprüche 1 bis 10 oder eines pharmazeutisch annehmbaren Derivats oder Salze derselben und einen pharmazeutisch annehmbaren Träger.

## Revendications

1. Nouveau pyrrolo[2,1-c][1,4]benzodiazépine de formule VIII, dans laquelle n₁ = 2, 3, 4, n₂=2,3,4.

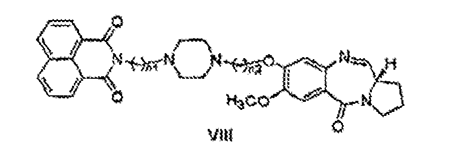

2. Composé selon la revendication 1, étant le 7-méthoxy-8-{2-[4-[2-(1,3-dioxobenz[de]isoquinoline-2-yl)alkyl]pipérazine-1-yl]alkyl}-oxy-(llaS)-1,2,3,11a tétrahydro-5H-pyrrolo [2,1-c][1,4]benzodiazépine-5-one de formule VIII, dans laquelle alkyl = éthyl, propyl, butyl.

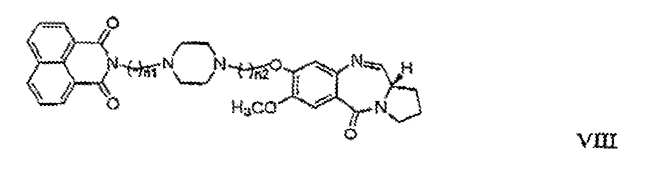

3. Composé selon la revendication 1, étant le 7-méthoxy-8-{2-[4-[2-(1,3-dioxobenz[de]isoquinoline-2-yl)éthyl]pipérazine-1-yl]éthyl]-oxy-(11aS)-1,2,3,11a tétrahydro-5H-pyrrolo [2,1-c][1,4]benzodiazépine-5-one de formule structurelle ci-dessous.

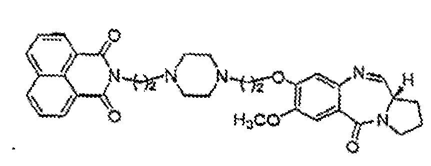

4. Composé selon la revendication 1, étant le 7-méthoxy-8-{3-[4-[2-(1,3-dioxobenz[de]isoquinoline-2-yl)éthyl]pipérazine-1-yl]propyl}-oxy-(11aS)-1,2,3,11a tétrahydro -5H-pyrrolo [2,1-c][1,4]benzodiazépine-5-one de formule structurelle ci-dessous.

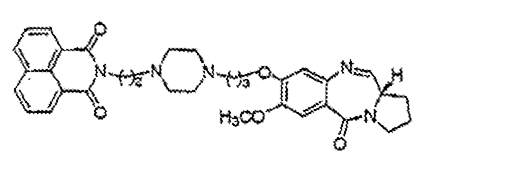

5. Composé selon la revendication 1, étant le 7-méthoxy-8-{4-[4-[2-(1,3-dioxobenz[de]isoquinoline-2-yl)éthyl]pipérazine-1-yl]butyl}-oxy-(11aS)-1,2,3,11a tétrahydro-5H-pyrrolo [2,1-c] [1,4]benzodiazépine-5-one de formule structurelle ci-dessous.

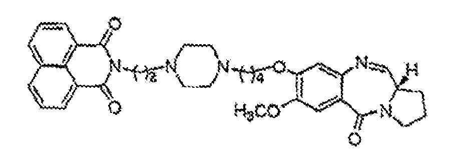

6. Composé selon la revendication 1, étant le 7-méthoxy-8-{3-[4-[3-(1,3-dioxobenz[de]isoquinoline-2-yl)propyl]pipérazine-1-yl]propyl}-oxy-(11aS)-1,2,3,11a tétrahydro-5H-pyrrolo [2,1-c][1,4]benzodiazépine-5-one de formule structurelle ci-dessous.

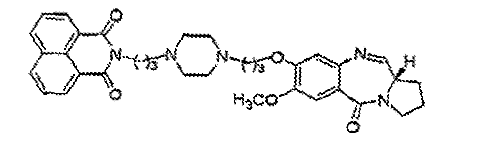

7. Composé selon la revendication 1, étant le 7-méthoxy-8-{4-[4-[4-(1,3-dioxobenz[de]isoquinoline-2-yl)butyl]pipérazine-1-yl]butyl}-oxy-(11aS)-1,2,3,11a tétrahydro-5H-pyrrolo [2,1-c][1,4]benzodiazépine-5-one de formule structurelle ci-dessous.

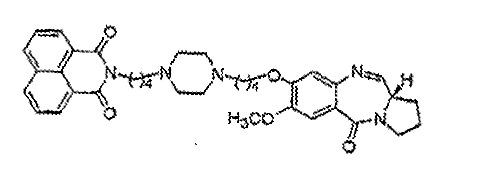

8. Composé selon la revendication 1, étant le 7-méthoxy-8-14-[4-[2-(1,3-dioxobenz[de]isoquinoline-2-yl)propyl]pipérazine-1-yl]butyl}-oxy-(11aS)-1,2,3,11a tétrahydro - 5H-pyrrolo [2,1-c][1,4]benzodiazépine-5-one de formule structurelle ci-dessous.

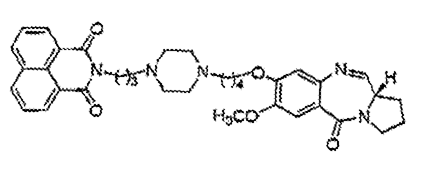

9. Composé selon la revendication 1, étant le 7-méthoxy-8-{3-[4-[2-(1,3-dioxobenz[de]isoquinoline-2-yl)butyl]pipérazine-1-yl]propyl}-oxy-(11aS)-1,2,3,11a tétrahydro -5H-pyrrolo [2,1-c][1,4]benzodiazépine-5-one de formule structurelle ci-dessous.

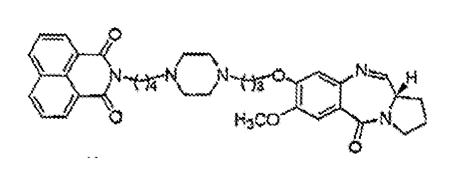

10. Composé selon la revendication 1, étant le 7-méthoxy-8-{3-[4-[3-(1,3-dioxobenz[de]isoquinoline-2-yl)propyl]pipérazine-1-yl]éthyl}-oxy-(11aS)-1,2,3,11a tétrahydro -5H-pyrrolo [2,1-c][1,4]benzodiazépine-5-one de formule structurelle ci-dessous.

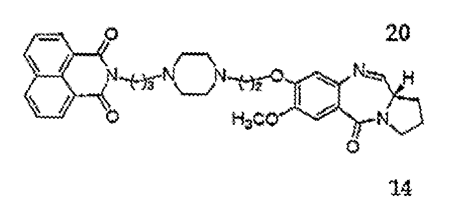

11. Procédé de préparation de pyrrolo[2,1-c][1,4]benzodiazépine de formule VIII 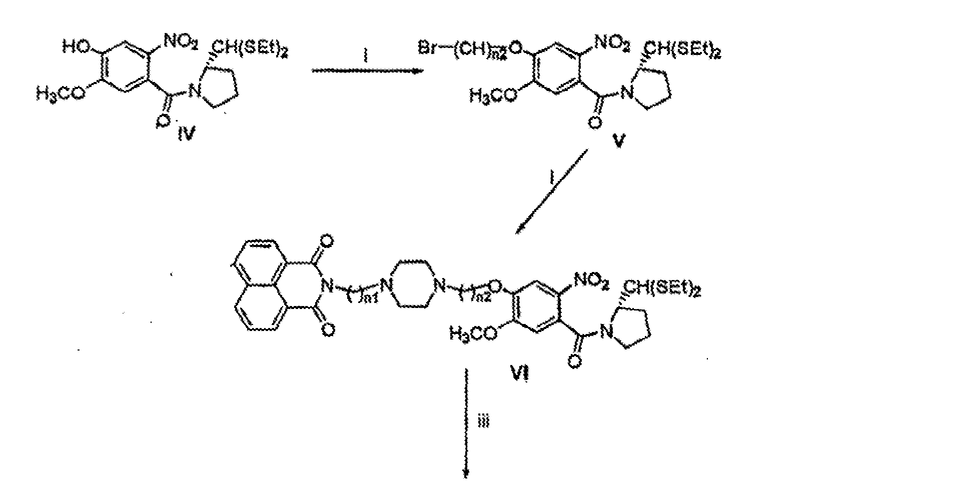 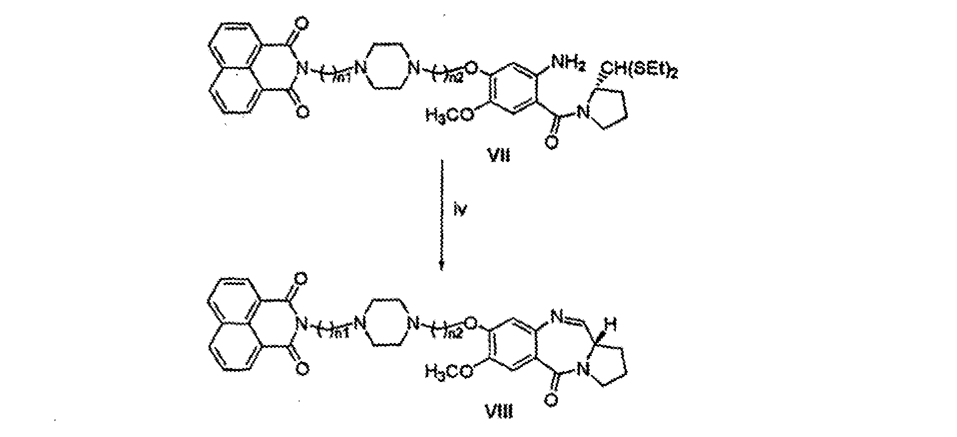 dans lequel n₁ = 2, 3, 4, n₂ = 2, 3, 4, comprenant la mise en réaction de (2S)-N-[4-hydroxy-5-méthoxy-2-nitrobenzoyl]pyrrolidine-2-carboxaldéhyde diéthyl thioacétal de formule IV avec des dibromoalcanes, dans un solvant miscible dans l'eau, aprotique, en présence d'une base non organique douce, à une température de reflux, pendant une durée de 48 h, l'isolation du (2S)-N-[4-(n-bromoalkoxy)-5-méthoxy-2-nitrobenzoyl]pyrrolidine -2-carboxaldéhyde diéthyl thioacétyl de formule IV, la mise en réaction du composé de formule V avec de la naphtalimide attachée à de la pipérazine, en présence de bases non organiques douces, l'isolation du composé de formule VI, sa réduction avec SnCl₂.2H₂O en présence de solvant organique, à une température de reflux, la réaction du composé amino ci-desssus, de formule VII, avec un agent de déprotection, pour obtenir la pyrrolo[2,1-c][1,4]benzodiazépine de formule VIII, dans laquelle n est tel qu'indiqué ci-dessus.

12. Composé selon l'une quelconque des revendications 1 à 10, ou un dérivé pharmaceutiquement acceptable, ou un sel de celui-ci, pour utilisation dans le traitement du cancer.

13. Utilisation d'un composé selon l'une quelconque des revendications 1 à 10, ou d'un dérivé pharmaceutiquement acceptable, ou d'un sel de celui-ci, dans la préparation d'un médicament pour le traitement du cancer.

14. Composition pharmaceutique comprenant une quantité pharmaceutiquement efficace d'un composé selon l'une quelconque des revendications 1 à 10, ou d'un dérivé pharmaceutiquement acceptable, ou d'un sel de celui-ci, et un excipient pharmaceutiquement acceptable.
